## Europäisches Patentamt
## European Patent Office
## Office européen des brevets

(19)

(11) Numéro de publication: **0 045 100 B1**

(12)

# FASCICULE DE BREVET EUROPEEN

(45) Date de publication du fascicule du brevet:
**14.03.84**

(21) Numéro de dépôt: **81200692.2**

(22) Date de dépôt: **19.06.81**

(51) Int. Cl.³: **C 07 B 27/00**, C 07 D 307/36 //
C07C43/23, C07C43/215,
C07C41/30, C07C15/44,
C07C11/02, C07C1/34,
C07C79/10, C07C76/02

(54) **Procédé de transformation d'un aldéhyde en un alcène, en particulier de furfural en furfurylidènes.**

(30) Priorité: **28.07.80 FR 8016767**

(43) Date de publication de la demande:
**03.02.82 Bulletin 82/5**

(45) Mention de la délivrance du brevet:
**14.03.84 Bulletin 84/11**

(84) Etats contractants désignés:
**AT BE CH DE GB IT LI LU NL SE**

(56) Documents cités:
**TETRAHEDRON LETTERS, juillet 1974, no. 30, Pergamon Press, GB TAGAKI, W. et al. "Wittig reaction in benzene-aqueous alkaline solution" pages 2587-2590**
**CHEMICAL ABSTRACTS, vol. 92, no. 1, January 7, 1980, page 569, abstract 6038q, New York, US**
**SYNTHESIS, juin 1976, Stuttgart, DE, M. MIKOLAJCZYK et al. "Horner-Wittig reactions in a two-phase system in the absence of a typical phase-transfer catalyst" pages 396-398**
**SYNTHESIS, novembre 1979, Stuttgart, DE, F. TEXIER-BOULLET et al. "The Wittig-Horner reaction on solid-liquid two phase systems", pages 884-885**

(73) Titulaire: **AGRIFURANE S.A., Z.I. de Boé Cédex 4412, F-47240 Bon Encontre (FR)**

(72) Inventeur: **Delmas, Michel, Rouens, F-12140 Entraygues (FR)**
Inventeur: **Gaset, Antoine, 75, allée de Brienne, F-31000 Toulouse (FR)**
Inventeur: **Le Bigot, Yves, Le Pous, F-34380 Saint Martin de Londres (FR)**

(74) Mandataire: **Barre, Philippe, Cabinet Barre, Gatti, Laforgue 95, rue des Amidonniers, F-31069 Toulouse Cedex (FR)**

BUNDESDRUCKEREI BERLIN

## Procédé de transformation d'un aldéhyde en un alcène, en particulier de furfural en furfurylidènes

L'invention concerne un procédé de transformation d'un aldéhyde en un alcène correspondant. Elle s'applique en particulier pour la transformation de furfural en furfurylidènes. Par aldéhyde, on entend de façon générale tout corps ayant une ou plusieurs fonctions aldéhydes. De plus, dans la suite, on entend de façon classique par »fonctionnalisé« la substitution, sur un carbone donné, d'un hydrogène par un radical organique; la position $\alpha$ est attribuée de façon habituelle au premier carbone de la chaîne considérée, qui est rattaché au carbone de la double liaison éthylénique provenant du sel de phosphonium; la position $\beta$ est attribuée au deuxième carbone de cette chaîne. Ces conventions sont adoptées par la plupart des spécialistes du domaine concerné.

La réaction de WITTIG découverte en 1954 est actuellement très utilisée pour préparer des alcènes à partir des aldéhydes correspondants; en particulier cette réaction permet d'obtenir des alcènes fonctionnalisés ou non en $\alpha$ ou $\beta$ d'un carbone de la double liaison éthylénique par un cycle ou une chaîne aliphatique, et non fonctionnalisé à partir du même carbone par un hétéroatome ou un groupe fonctionnel hétéroatomique; on sait que ce type d'alcènes intervient dans de nombreuses synthèses où sont euxmêmes directement actifs dans de nombreux domaines notamment dans l'industrie chimique (polymères, intermédiaires de synthèse ...), pharmaceutique (antibactériens, analgésiques ...), agrochimique et agrolimentaire (phéromones, parfums, insecticides ...). Les furfurylidènes sont particulièrement actifs dans ce dernier domaine, essentiellement par leurs propriétés antibactériennes et antifongiques.

La réaction de WITTIG consiste en une condensation entre un sel de phosphonium et un composé carbonylé en présence de certaines bases fortes et en milieu strictement anhydre; les bases utilisées sont des bases fortes du type butyl-lithium, hydrures ... qui décomposent l'eau (force basique supérieure à celle de l'ion hydroxyde). L'obtention d'alcènes par cette réaction pose des problèmes techniques très difficiles à résoudre à échelle industrielle. En premier lieu, l'état strictement anhydre du milieu réactionnel est difficile à atteindre dans les installations industrielles et exige des traitements et contrôles spéciaux de mise en œuvre très onéreuse (étuvage, séchage par courant d'azote ...). En outre les bases fortes l'eau, qui rend leur stockage dangereux; de plus, ces produits sont chers et grèvent considérablement le coût de la synthèse. Au surplus, la réaction est généralement mise en œuvre dans des solvants lourds (tels que diméthylsulfoxyde DMSO, diméthylformamide DMF, héxaméthylphosphortriamide (HMPT) ...), qui sont difficile à séparer des produits réactionnels et à recycler. Enfin, cette réaction qui est opérante avec un rendement satisfaisant avec les aldéhydes aromatiques, devient peu intéressante avec les aldéhydes aliphatiques en raison de leur faible rendement.

En vue d'éliminer ou de réduire ces défauts, la réaction de WITTIG a fait l'objet de nombreuses études et deux types essentiels de techniques de mise en œuvre ont été développés. L'une des techniques appliquée vers 1975 à la réaction de WITTIG est celle de la catalyse par transfert de phase (on se reportera par exemple à la publication suivante qui décrit ce type de technique: TETRAHEDRON LETTERS n° 30, 1974, PERGAMON PRESS GB — auteurs: W. TAGAKI et al.); cette technique permet d'éviter les difficultés liées à l'état anhydre imposé au milieu, mais implique la présence d'une phase aqueuse importante et corrosive qui limite son intérêt; de plus, le rendement en alcène est médiocre et la réaction n'est pas stéréosélective (rapport Z/E de l'ordre de 1). Enfin, cette technique n'est pas applicable aux aldéhydes aliphatiques en raison de réactions secondaires d'aldolisation qui apparaissent; cette limitation est un inconvénient grave en pratique car les alcènes aliphatiques sont des substrats extrêmement utiles, qui sont indispensables pour la synthèse de produits actifs, essentiels dans le domaine de la chimie biologique, tels par exemple que les phéronomes et prostaglandines.

Un autre type de technique utilisant une réaction dite de WITTIG—HORNER, dérivée de la réaction de WITTIG, consiste à faire réagir un phosphonate en présence d'une base forte en milieu liquide biphasique, constitué par une phase aqueuse et une phase organique (on peut se reporter pour plus de précision à la publication suivante: revue SYNTHESIS, 1976, 396, auteurs: M. MIKOLAJCZYK et al.). Une variante décrite en 1979 (revue SYNTHESIS, 1979, auteurs: FOUCAULT et al.) consiste à réaliser la réaction en phases solide/liquide avec un agent de transfert pour accroître la vitesse de transformation. Toutefois, comme la technique précédente et pour les mêmes raisons, cette technique n'est pas applicable aux aldéhydes aliphatiques, ce qui représente une grave limitation comme déjà mentionné. De plus,la réaction mise en jeu qui ne peut être menée qu'avec un phosphonate (et non un sel de phosphonium) permet d'obtenir essentiellement des alcènes fonctionnalisés en $\alpha$ ou $\beta$ du carbone de la double liaison éthylénique provenant des phosphonates, par un hétéroatome ou un groupe fonctionnel hétéroatomique. Ces alcènes sont très différents sur le plan de leur activité des alcènes évoqués précédemment (on entend de façon classique par »hétéroatome« tout atome différent du carbone et de l'hydrogène s'insérant dans une combinaison organique, et par »groupe fonctionnel hétéroatomique« toute combination organique comportant un ou plusieurs hétéroatomes).

La présente invention se propose d'indiquer un nouveau procédé de transformation d'un aldéhyde en alcène du type dans lequel l'aldéhyde est mis en présence d'un réactif phosphorique et d'une base, en solution dans un solvant organique aprotique.

Un objectif de l'invention est en particulier d'indiquer un procédé de ce type, susceptible d'être mis en œuvre avec de rendements satisfaisants aussi bien avec des aldéhydes aromatiques ou hétéroaromatiques qu'avec des aldéhydes aliphatiques, pour obtenir le type d'alcènes sus-evoqués.

Un autre objectif est de suprimer l'exigence stricte du caractère anhydre de la réaction de WITTIG en fournissant un procédé apte à être mis en œuvre en présence d'eau dans des conditions très performantes.

Un autre objectif est de permettre l'utilisation de bases classiques ne présentant aucun risque et bénéficiant de coûts réduits.

Un autre objectif de l'invention est d'indiquer un procédé apte à opérer avec un solvant léger, peu onéreux, suspectible d'être régénéré et duquel les produits de synthèse sont faciles à séparer.

Un autre objectif est d'indiquer un procédé mettant en œuvre une réaction non exothermique, ne présentant aucun danger, même avec de grandes quantités de produits en présence, et se déroulant dans des conditions douces de température et de pression.

Un autre objectif est d'indiquer un procédé conduisant à une bonne stéréosélectivité.

A cet effet, le procédé de transformation visé par l'invention est du type dans lequel l'aldéhyde est mis en présence d'un sel de phosphonium et d'une base, en solution dans un solvant organique aprotique; selon la présente invention, on réalise la réaction dans les conditions suivantes:

— on utilise une base minérale ayant en milieu aqueux une force basique inférieure ou égale à celle de l'ion hydroxyde ou une base organique fortement basique de la famille des composés azotés, notamment pyridine et triethylamine,
— on ajuste le taux d'hydratation du milieu réactionnel organique de façon à avoir dans ce milieu entre environ 0,5 et 5 moles d'eau par mole d'aldéhyde.

Ainsi, l'originalité du procédé de transformation conforme à l'invention réside dans le fait que la réaction de transformation de l'aldéhyde s'effectue avec un sel de phosphonium en milieu organique avec un taux d'hydradatation faible, contrôlé de façon précise.

Il est essentiel de remarquer que l'aldéhyde, le réactif phosphorique et également la base ne se trouvent pas en solution aqueuse; dans le cas d'une base minérale celle-ci se trouve à l'état solide, l'aldéhyde et le sel de phosphonium étant en solution dans le solvant organique; dans le cas d'une base organique de la famille de amines, les trois corps sont en solution dans le solvant organique. On a donc de toute façon une seule phase liquide organique avec un taux d'hydratation faible et contrôlé.

Les expérimentation sont montré que la maîtrise de ce taux d'hydratation dans la plage ci-dessus définie permet, en utilisant une base du groupe indiqué, une transformation sélective de l'aldéhyde en alcène avec de très hauts rendements. Ce taux d'hydratation est facile à contrôler et les conditions difficiles de la réaction de WITTIG sont ainsi éliminées. Ce taux d'hydratation peut être préférentiellement ajusté aux environs de 2 moles d'eau par mole d'aldéhyde, ce taux conduisant de façon nette à un rendement maximum.

De plus, la base utilisée peut être une base classique notamment un composé ionique du potassium où l'anion présente un caractère basique (hydroxyde de potassium, carbonate de potassium, phosphate de potassium, perchlorate de potassium, ...); ce type de base n'entraîne aucun risque sur le plan industriel et son prix de revient est faible.

En outre, on a pu constater que cette réaction est également opérante avec de bons rendements avec les aldéhydes aliphatiques. Les conditions du procédé permettent l'inhibition des réactions secondaires d'aldolisation, ce qui explique que les rendements obtenus avec ce type d'aldéhyde présent également des valeurs élevées.

Il est à noter que, dans le cas des aldéhydes aromatiques, il se produit parfois dans les procédés classiques une réaction secondaire (dite de CANNIZZARO) qui limite le rendement en alcène. Dans le cas du procédé de l'invention, cette réaction est extrêmement peu sensible; elle est totalement inhibée lorsqu'on utilise comme base le carbonate de potassium.

La réaction se produisant dans le procédé de l'invention est légèrement endothermique et ne suscite aucun risque de développement brutal. Le rendement est amélioré par un léger chauffage; en pratique, le milieu réactionnel est de préférence porté à une température approximativement comprise entre 40° C et 110° C.

Les solvants utilisés sont avantageusement des solvants organiques, non basiques ou peu basiques tels par exemple que dioxanne, diméthoxyétane (DME), chlorure de méthylène. Ces solvants améliorent généralement le rendement de la réaction et permettent une séparation facile des alcènes par distillation. Il sont peu onéreux et sont très faciles à régénérer.

Certains solvants tels que les solvants oxygénés ou aromatiques conduisent à une réaction très stéréosélective, l'alcène Z étant obtenu majoritairement. D'autres solvants tels que le chlorure de méthylène, conduisent à une réaction non stéréosélective de sorte que le procédé de l'invention permet d'orienter la stéréochimie de la réaction selon l'application envisagée, par un choix approprié du solvant.

En outre, on a pu constater qu'il était intéressant pour accroître le rendement d'ajuster la quantité de solvant de façon à avoir une dilution de l'aldéhyde comprise entre 0,2 et 2 moles par litre de solvant.

De plus, sur le plan des quantités relatives de produits, on opère avantageusement dans les conditions suivantes:

— base utilisée en léger excès stoechiométrique par rapport à l'aldéhyde,
— sel de phosphonium utilisé en léger excès stoechiométrique par rapport à l'aldéhyde.

Dans le cas fréquent où l'on désire obtenir un alcène non fonctionnalisé en $\alpha$ ou $\beta$ de la double liaison éthylénique, on utilisera de préférence un sel de phosphonium obtenu à partir de triphénylphosphine et d'un halogénure R CH$_2$X où X est un ion halogénure (Cl, Br, I) et R peut être une chaîne aliphatique ou un cycle saturé ou insaturé. Ces alcènes on un intérêt pratique, large et varié en agrochimie, dans les industries pharmaceutiques et chimiques, tant par leurs activités biologiques propres que comme intermédiaires de synthèse; c'est un avantage important de l'invention que de permettre d'orienter la réaction vers la production de ce type de composé.

L'invention exposée de façon générale ci-dessus est illustrée ci-après par de exemples non limitatifs de mise en œuvre.

Exemple 1

Synthèse de vinyl-2 furane à partir de furanne-carboxaldéhyde en présence de carbonate de potassium et de bromure de méthyltriphényl phosphonium dans le nitrobenzène.

Dans un réacteur de 250 ml équipé d'un réfrigérant, d'un agitateur mécanique et d'une ampoule à addition, on place 0,025 mole de sel de phosphonium, 0,025 mole de carbonate de potassium, 0,02 mole d'aldéhyde et 0,04 mole d'eau dans 20 cm$^3$ de nitrobenzène.

On chauffe progressivement la solution jusqu'à 90°C pendant 6 heures. Le mélange réactionnel est ensuite refroidi à la température ambiante. La majeure partie de l'oxyde de triphénylphosphine formé précipite et est séparé par filtration de la phase organique qui est ensuite analysée par chromatographie en phase vapeur sur colonne du type 0V101. Après séchage de la phase liquide organique sur sulfate de magnésium, le vinyl-2 furanne formé est obtenu avec un rendement voisin de 90% après distillation. Le solvant est ensuite récupéré et recyclé.

Le vinyl-2 furanne est caractérisé par ses spectres infra-rouge et de résonance magnétique nucléaire du proton et du carbone 13 (R.M.N.) dont les résultats sont confirmés par microanalyse.

Le composé de départ: le furanne-carboxaldéhyde-2 (ou furfural) est fabriqué industriellement à partir de sous-produits agricoles riches en pentosanes (rafles de maïs balles de riz et d'avoine, bagasses de canne à sucre, coques d'arachide et déchets de bois). Ces déchets traités en milieu acide concentré conduisent à des aldopentoses obtenus par hydrolyse de leurs pentosanes qui se déshydratent pour aboutir au furfural.

Exemple 2

Synthèse de pentène-1,yl-2 furanne à partir du furanne-carboxaldéhyde-2 en présence de carbonate de potassium et de bromure de butyl-triphényl phosphonium dans le diméthoxy-1,2 éthane.

Dans un réacteur identique de 250 ml, on place 0,025 mole de sel de phosphonium, 0,025 mole de carbonate de potassium, 0,02 mole d'aldéhyde et 0,04 mole d'eau dans 20 cm$^3$ de diméthoxy-1,2 éthane.

Le milieu est mis en agitation soit pendant:

— 3 heures au reflux,
— 6 heures à 80°C,
— 12 heures à 60°C.

Ces conditions permettent la transformation complète du furfural en furfurylidène-2 butane qui est extrait après filtration, séchage et concentration du milieu réactionnel par chromatographie sur une courte colonne de silicagel en utilisant l'hexane comme éluant.

Exemple 3

Synthèse de l'hydroxyl-1, méthoxy-2, pentène-1,yl-4 bensène à partir de la vanilline en présence de carbonate de potassium et de bromure de butyl-triphényl phosphonium dans le dioxanne-1,4.

On place dans un réacteur de 1 litre 0,12 mole de sel de phosphonium, 0,12 mole de carbonate de potassium, 0,1 mole d'aldéhyde et 0,2 mole d'eau dans 100 cm$^3$ de dioxanne-1,4.

Après 16 heures de réaction au reflux du dioxanne-1,4, le milieu réactionnel est filtré, séché et concentré. L'alcène formé est obtenu après distillation (p.eb = 90°/0,5 mm Hg) avec un rendement voisin de 70% et caractérisé par ses spectres infra-rouge et R.M.N.

La réaction est stéréosélective et l'isomère Z est obtenu majoritairement (% isomère Z/% isomère E: 85/15).

Les aldéhydes phénoliques qui peuvent être ainsi valorisés sont pour la plupart extraits de la matière végétale notamment par traitement biologique ou chimique de la lignine.

Exemple 4

Synthèse de l'anéthole à partir de l'anisaldéhyde en présence de carbonate de potassium et de bromure d'éthyltriphényl phosphonium dans le dioxanne-1,4.

On mélange dans un réacteur 0,025 mole de sel de phosphonium, 0,025 mole de carbonate de potassium, 0,02 mole d'aldéhyde et 0,04 mole d'eau dans 20 cm³ de dioxanne-1,4.

Le mélange réactionnel est agité au reflux du dioxanne-1,4 pendant douze heures. L'anéthole (% isomère Z/% isomère E: 87/12) est obtenu pur avec un rendement de l'ordre de 85% et identifié par comparaison au produit commercial et analyse de ses spectres infra-rouge et R.M.N.

## Exemple 5

Synthèse du pentène-lyl-1 benzène à partir du benzaldéhyde en présence d'hydroxyde de sodium et de chlorure de butyl-triphényl phosphonium dans le chlorure de méthylène.

On place dans un réacteur 0,025 mole de sel de phosphonium, 0,025 mole d'hydroxyde de sodium, 0,02 mole d'aldéhyde et 0,04 mole d'eau dans 20 cm³ de chlorure de méthylène.

Après 10 heures de réaction au reflux du solvant, l'alcène est obtenu pur avec un rendement de 90% dosé après extraction du milieu par chromatographie sur colonne. La réaction dans le chlorure de méthylène n'est plus stéréosélective (% isomère Z/% isomère E: 50/50).

## Exemple 6

Synthèse du divinylbenzène à partir de l'aldéhyde téréphtalique en présence de carbonate de potassium et de bromure de méthyl-triphényl phosphonium dans le dioxanne-1,4.

On mélange dans un réacteur 0,05 mole de sel de phosphonium, 0,05 mole de carbonate de potassium, 0,02 mole d'aldéhyde et 0,1 mole d'eau dans 30 cm³ de dioxanne-1,4.

Le milieu réactionnel est agité au reflux du dioxanne-1,4 pendant 12 heures. Le divinylbenzène est obtenu avec un rendement de 85% et identifié par comparaison de ses caractéristiques physicochimique à celle du produit commercial.

## Exemple 7

Synthèse du décène-5 à partir de l'hexanal en présence de carbonate de potassium et de bromure de butyl-triphényl phosphonium dans le dioxanne-1,4.

On mélange dans un réacteur 0,025 mole de sel de phosphonium, 0,025 mole de carbonate de potassium, 0,02 mole d'aldéhyde et 0,04 mole d'eau dans 20 cm³ de dioxanne-1,4.

Le mélange réactionnel est porté au reflux et maintenu sous agitation pendant 4 heures. Le décène-5 est obtenu après chromatographie sur colonne avec un rendement de 80% et identifié et caractérisé par comparaison avec le produit commercial.

Il faut souligner que dans ces conditions expérimentales, la réaction secondaire d'aldolisation est inhibée.

## Exemple 8

Synthèse d'une phéromone de l'insecte »Diptera-Musca domestica« à partir d'octanal et de bromure de pentadécanyl-triphényl phosphonium en présence de carbonate de potassium dans le dioxanne-1,4.

On mélange dans un réacteur 0,025 mole de sel de phosphonium, 0,025 mole de carbonate de potassium, 0,02 mole d'aldéhyde et 0,04 mole d'eau dans 20 cm³ de dioxanne-1,4.

Le milieu réactionnel est porté au reflux et maintenu sous agitation pendant 10 heures. L'alcène correspondant est obtenu après chromatographie sur colonne avec un rendement de 70% et caractérisé par ses spectres infra-rouge et de résonance magnétique nucléaire et par microanalyse.

On peut de cette façon obtenir un grand nombre de phéromones. A titre d'exemple, en utilisant l'aldéhyde myristique et le même sel de phosphonium, on obtient la phéromone de l'insecte »Diptera-Musca automnalis« (phéromone obtenue: Z-14 nonacosène).

## Exemple 9

Synthèse du p-nitrostyrène à partir du formaldéhyde polymérisé et du bromure de p-nitrobenzyltriphénil phosphonium en présence de carbonate de potassium dans le dioxanne-1,4.

Dans un réacteur de 250 ml on place 0,025 mole de sel de phosphonium, 0,025 mole de carbonate de potassium, 0,02 mole de formaldéhyde (trioxane ou paraformaldéhyde) et 0,04 mole d'eau dans 20 ml de dioxanne-1,4.

Après deux heures de réaction à 80°C le milieu réactionnel est refroidi à la température ambiante, filtré, séché sur sulfate de magnésium. On fait ensuite percoler la solution concentrée en présence d'hexane comme éluant sur une courte colonne de silicagel. Le p-nitro-styrène (p.f. = 29°C) est obtenu pur avec un rendement de 90%.

Il faut noter que pour tous ces exemples, l'augmentation de la quantité de base ne modifie pas sensiblement la réactivité, ce qui semble indiquer que le rôle de l'eau serait de favoriser la décomposition de l'adduct intermédiaire.

## Revendications

Procédé de transformation d'un aldéhyde en un alcène corrrespondant, dans lequel l'aldéhyde est mis en présence d'un sel de phosphonium et d'une base, en solution dans un solvant organique aprotique, ledit procédé étant caractérisé en ce que l'on réalise la réaction dans les conditions suivantes:

— on utilise une base minérale ayant en milieu aqueux une force basique inférieure ou égale à celle de l'ion hydroxyde ou une base organique fortement basique de la famille des composés azotés,

— on ajuste le taux d'hydratation du milieu réactionnel organique de façon à avoir dans ce milieu entre environ 0,5 et 5 moles d'eau par mole d'aldéhyde.

2. Procédé de transformation selon la revendication 1, caractérisé en ce que l'on ajuste le taux d'hydratation du milieu réactionnel aux environs de 2 moles d'eau par mole d'aldéhyde.

3. Procédé de transformation selon l'une des revendications 1 ou 2, caractérisé en ce que l'on dispose la base dans le milieu réactionnel sous forme d'un composé ionique solide de potassium où l'anion présente un caractère basique, avec addition d'eau appropriée pour atteindre le taux d'hydratation ci-dessus évoqué.

4. Procédé de transformation selon l'une des revendications 1, 2 ou 3, caractérisé en ce que la base est mise en présence en léger excès stoechiométrique par rapport à l'aldéhyde.

5. Procédé de transformation selon l'une des revendications 1, 2, 3 ou 4, caractérisé en ce que la réaction est réalisée en solution un solvant organique, non basique ou peu basique.

6. Procédé de transformation selon la revendication 5, caractérisé en ce que le solvant utilisé est du dioxanne ou du diméthoxyéthane (D.M.E.), ou du chlorure de méthylène ou du nitrobenzène.

7. Procédé de transformation selon l'une des revendications 5 ou 6, caractérisé en ce que la quantité de solvant est ajustée de façon à réaliser une dilution de l'aldéhyde comprise entre 0,2 et 2 moles par litre de solvant.

8. Procédé de transformation selon l'une des revendications 1, 2, 3, 4, 5, 6 ou 7, caractérisé en ce que l'on utilise comme sel de phosphonium un sel obtenu à partir de triphénylphosphine et d'un halogénure R CH$_2$ X où X est un ion halogénure (Cl, Br, I) et R est une chaîne aliphatique ou un cycle saturé ou insaturé.

9. Procédé de transformation selon l'une des revendications 1, 2, 3, 4, 5, 6, 7 ou 8, dans lequel le sel de phosphonium est mis en présence en léger excès stoechiométrique par rapport à l'aldéhyde.

10. Procédé de transformation selon l'une des revendications 1, 2, 3, 4, 5, 6, 7 ou 8, caractérisé en ce que le milieu réactionnel est porté à une température comprise entre 40°C et 110°C.

11. Procédé de transformation selon l'une des revendications 1, 2, 3, 4, 5, 6, 7, 8, 9 ou 10, pour la transformation du furfural en furfurylidènes, caractérisé en ce que l'on utilise comme base du K$_2$CO$_3$ et comme solvant le dioxanne ou le nitrobenzène.

## Patentansprüche

1. Verfahren zum Umsetzen eines Aldehyds in ein entsprechendes Alken, bei welchem das Aldehyd mit einem Phosphoniumsalz und einer Base, in einem aprotischen organischen Lösungsmittel gelöst, zusammengebracht wird, wobei besagtes Verfahren dadurch gekennzeichnet ist, daß man die Reaktion unter den folgenden Bedingungen durchführt:

— man verwendet eine anorganische Base mit einer basischen Stärke in wäßrigem Medium, welche geringer als die des Hydroxylions oder ihr gleich ist, oder eine stark basische organische Base aus der Gruppe der Stickstoffverbindungen;
— man stellt den Wassergehalt des organischen Reaktionsmediums so ein, daß in diesem Medium zwischen etwa 0,5 und 5 Mol Wasser pro Aldehyd vorliegt.

2. Umsetzungsverfahren nach Anspruch 1, dadurch gekennzeichnet, daß man den Wassergehalt des Reaktionsmediums auf annähernd 2 Mol Wasser pro Mol Aldehyd einstellt.

3. Umsetzungsverfahren nach einem der Ansprüche 1 oder 2, dadurch gekennzeichnet, daß man die Base in das Reaktionsmedium in Form einer festen ionischen Kaliumverbindung einführt, in welcher das Anion eine basische Natur aufweist, mit der entsprechenden Zugabe von Wasser, um den vorerwähnten Wassergehalt zu erreichen.

4. Umsetzungsverfahren nach einem der Ansprüche 1, 2 oder 3, dadurch gekennzeichnet, daß die Base in geringem stöchiometrischem Überschuß im Verhältnis zum Aldehyd eingeführt wird.

5. Umsetzungsverfahren nach einem der Ansprüche 1, 2, 3 oder 4, dadurch gekennzeichnet, daß die Reaktion in Lösung in einem nichtbasischen oder wenig basischen organischen Lösungsmittel durchgeführt wird.

6. Umsetzungsverfahren nach Anspruch 5, dadurch gekennzeichnet, daß das verwendete Lösungsmittel Dioxan oder Dimethoxyäthan (DMÄ) oder Methylenchlorid oder Nitrobenzol ist.

7. Umsetzungsverfahren nach einem der Ansprüche 5 oder 6, dadurch gekennzeichnet, daß die Lösungsmittelmenge so eingestellt wird, daß eine Verdünnung des Aldehyds zwischen 0,2 und 2 Mol pro Liter Lösungsmittel zustande gebracht wird.

8. Umsetzungsverfahren nach einem der Ansprüche 1, 2, 3, 4, 5, 6 oder 7, dadurch gekennzeichnet, daß man als Phosphoniumsalz ein aus Triphenylphosphin und einem Halogenid RCH$_2$X erhaltenes Salz verwendet, wobei X ein Halo-genidion (Cl, Br, J) und R eine aliphatische Kette oder ein gesättigter oder ungesättigter Ring ist.

9. Umsetzungsverfahren nach einem der Ansprüche 1, 2, 3, 4, 5, 6, 7 oder 8, bei welchem das Phosphoniumsalz in geringem stöchiometrischem Überschuß im Verhältnis zum Aldehyd eingeführt wird.

10. Umsetzungsverfahren nach einem der Ansprüche 1, 2, 3, 4, 5, 6, 7 oder 8, dadurch gekennzeichnet, daß das Reaktionsmedim auf eine Temperatur zwischen 40°C und 110°C gebracht wird.

11. Umsetzungsverfahren nach einem der Ansprüche 1, 2, 3, 4, 5, 6, 7, 8, 9 oder 10 zum Umsetzen von Furfural in Furfurylidene, dadurch gekennzeichnet, daß man $K_2CO_3$ als Base und Dioxan oder Nitrobenzol als Lösungsmittel verwendet.

## Claims

1. Process for the transformation of an aldehyde into a corresponding alkene, in which the aldehyde is brought into contact with a phosphonium salt and a base, in solution in an aprotic organic solvent, said process being characterised in that the reaction is carried out in the following conditions:

— an inorganic base, having a basic strength less than, or equal to, that of the hydroxyl ion in aqueous medium, or a strongly basic organic base from the group of nitrogen compounds is used;
— the water content of the organic reaction medium is adjusted so as to have between about 0.5 and 5 Moles, of water per Mole of aldehyde in the medium.

2. Transformation process according to Claim 1, characterised in that the water content of the reaction medium is adjusted to approximately 2 Moles of water per Mole of aldehyde.

3. Transformation process according to either Claim 1 or 2, characterised in that the base is placed in the reaction medium in the form of a solid ionic potassium compound, in which the anion is of basic nature, with the appropriate addition of water for reaching the abovementioned water content.

4. Transformation process according to one of the Claims 1, 2 or 3, characterised in that the base is introduced in slight stoichiometric excess, relative to the aldehyde.

5. Transformation process according to one of the Claims 1, 2, 3 or 4, characterised in that the reaction is carried out in solution in a non-basic or slightly basic organic solvent.

6. Transformation process according to Claim 5, characterised in that the solvent used is dioxan or dimethoxyethane (D.M.E.) or methylene chloride or nitrobenzene.

7. Transformation process according to either Claim 5 or 6, characterised in that the quantity of solvent is adjusted so as to bring about a dilution of the aldehyde between 0.2 and 2 Moles per litre of solvent.

8. Transformation processs according to one of the Claims 1, 2, 3, 4, 5, 6 or 7, characterised in that the phosphonium salt used is a salt obtained from triphenylphosphine and a halide $RCH_2X$, where X is a halide ion (Cl, Br, I) and R is an aliphatic chain or a saturated or unsaturated ring.

9. Transformation process according to one of the Claims 1, 2, 3, 4, 5, 6, 7 or 8, in which the phosphonium salt is introduced in slight stoichiometric excess, relative to the aldhyde.

10. Transformation process according to one of the Claims 1, 2, 3, 4, 5, 6, 7 or 8, characterised in that the reaction medium is taken to a temperature between 40°C and 110°C.

11. Transformation process according to one of the Claims 1, 2, 3, 4, 5, 6, 7, 8, 9 or 10 for the transformation of furfural into furfurylidenes, characterised in that the base used is $K_2CO_3$ and the solvent used is dioxan or nitrobenzene.